# EUROPÄISCHE PATENTSCHRIFT

(11) **EP 0 682 917 B1**
(45) Veröffentlichungstag und Bekanntmachung des Hinweises auf die Patenterteilung: **12.08.1998**
(21) Anmeldenummer: 95810267.5
(22) Anmeldetag: 22.04.1995
(51) Int. Cl.: A61B 17/58, A61B 17/16, A61B 17/17, B25B 23/10

(54) **Schraubwerkzeug für eine Schraube, bestehend aus einem Bolzenteil und einer darauf aufschraubbaren Mutter**
Screwdriver for a screw consisting of a bolt and a nut adapted to be screwed thereon
Outil de vissage pour une vis, consitant en un boulon et en un écrou qui peut être vissé sur ledit boulon

(30) Priorität: 22.04.1994 CH 1252/94
(43) Veröffentlichungstag der Anmeldung: 22.11.1995
(73) Patentinhaber: INSTITUT STRAUMANN AG, 4437 Waldenburg (CH)
(72) Erfinder: Mundwiler, Ulrich, CH 4456 Tenniken (CH); Gaab, Michael R., D 30625 Hannover (DE); Sutter, Franz, CH 4435 Niederdorf (CH); Merz, Beat R., CH 5000 Aarau (CH)
(74) Vertreter: Ullrich, Gerhard, Dr.

(56) Entgegenhaltungen:
- EP-A- 0 059 044
- EP-A- 0 369 603
- WO-A-91/09572
- BE-A- 551 875
- CH-A- 458 615
- CH-A- 621 476
- FR-A- 1 548 276
- US-A- 4 537 185
- US-A- 4 736 657

## Beschreibung

### Anwendung der Erfindung

Die Erfindung betrifft ein Schraubwerkzeug zum Eindrehen einer Schraube, bestehend aus einem Bolzenteil und einer separaten, auf das Bolzenteil aufschraubbaren Mutter. Die Schraube dient insbesondere zum Zusammenhalten von einem ersten Knochenfragment und einem zweiten Knochenfragment, z.B. bei einer Densfraktur des Axis, wie sie hauptsächlich bei Verkehrsunfällen geschehen kann.

### Stand der Technik

Es ist chirurgische Praxis, Knochenfragmente mittels Schrauben zu verbinden. Ein herkömmlicher Schraubentyp ist einstückig, wobei das Bolzenteil ein Gewinde aufweist und mit einem an der Spitze des Bolzenteils gegenüberliegenden Kopf abschliesst. Zum Eindrehen solcher Schrauben werden an sich bekannte Schraubwerkzeuge verwendet.

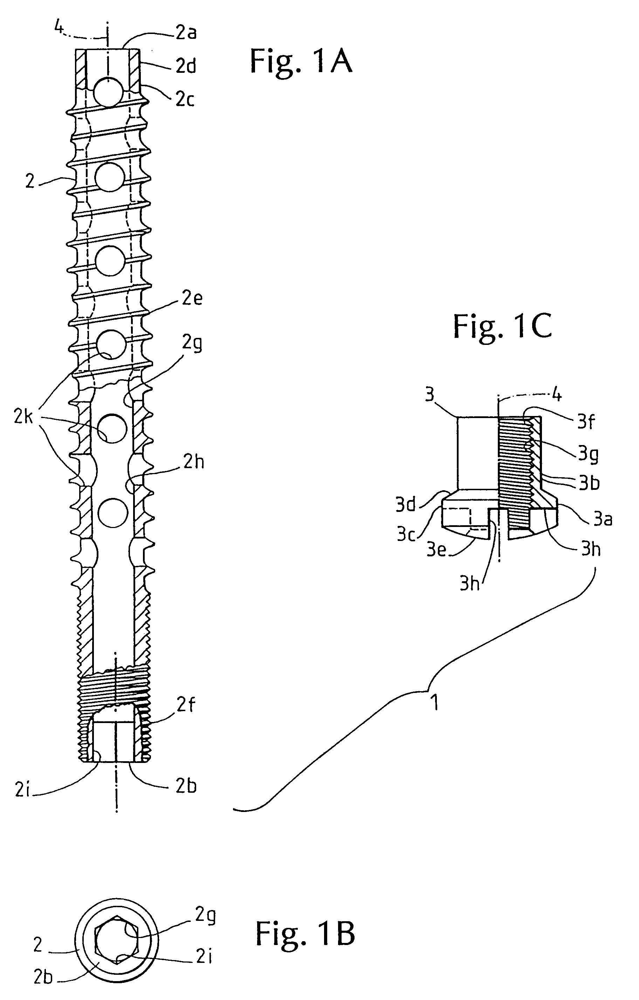

Ein weiterer Schraubentyp (vgl. Figuren 1A bis 1C) besteht aus einem Bolzenteil und einer separaten, auf das Bolzenteil aufschraubbaren Mutter. Durch beide Schraubenteile erstreckt sich eine Axialbohrung, so dass die Schraube auf einen zuvor gesetzten Kirschner-Draht aufgesteckt werden kann. Der Kirschner-Draht dient zuerst als Richtungsorientierung beim Erstellen der Schraubenbohrung; nach dem Eindrehen der Schraube wird dieser herausgezogen. Mittels der aufschraubbaren Mutter wird eine Kompression der beiden Knochenteile ermöglicht. Für das Eindrehen des Bolzenteils in die vorbereitete Aufnahmebohrung und das Aufschrauben der Mutter werden derzeit zwei verschiedene Werkzeuge benutzt. Das Erfordernis von zwei Eindrehwerkzeugen bedingt nicht nur ein umfangreicheres Instrumentarium, sondern der Chirurg muss nacheinander auch beide Eindrehwerkzeuge handhaben. Letztlich müssen die Werkzeuge nachbehandelt und aufbewahrt werden, so dass ein ziemlicher Aufwand entsteht.

### Aufgabe der Erfindung

Angesichts der Umstände, welche das bisherige Erfordernis von zwei Eindrehwerkzeugen, neben dem anderen nötigen Instrumentarium, verursachen, liegt der Erfindung die Aufgabe zugrunde, ein kombiniertes Eindrehwerkzeug zu schaffen, das sowohl zum Eindrehen des Bolzenteils als auch zum Aufschrauben der Mutter geeignet ist. Hierbei soll das Eindrehwerkzeug für besondere Anwendungen zur Führung auf einen gesetzten Kirschner-Draht aufsteckbar und in eine Gewebeschutzhülse einsetzbar sein. Ferner muss das Eindrehwerkzeug hohe Funktionssicherheit gewährleisten und eine unproblematische, sterile Reinigung ermöglichen.

### Wesen der Erfindung

Das Schraubwerkzeug ist als bifunktionales Instrument zum Schrauben eines Bolzen und eines darauf aufschraubbaren Kopfes konzipiert. Der Bolzen und der Kopf bilden zusammen die eine Schraube. Der Bolzen und der Kopf besitzen jeweils eine durchgängige Axialbohrung und Mitnehmerkonturen für den Eingriff des Schraubwerkzeugs. Die Mitnehmerkontur am Bolzen ist ein innerhalb der Axialbohrung nicht-rotationssymmetrischer, vieleckförmiger Abschnitt. Die Mitnehmerkontur am Kopf hat die Form radialer Einschnitte.

Das Bolzen- und Kopfteil, die alternativ in Funktion setzbar sind, bilden das Kombinations-Schraubwerkzeug. Das Bolzen- und Kopfteil weisen zuvorderst verschieden konturierte Arbeitsabschnitte auf, die dazu bestimmt sind, formschlüssig in Mitnehmerkonturen am Bolzen bzw. am Kopf einzugreifen und die Drehbewegung des Schraubwerkzeugs zu übertragen. Das Bolzenteil lässt sich teleskopisch in das Kopfteil einsetzen und mit einer Relativverschiebung zwischen beiden ist deren Mitnehmerfunktion wahlweise herstell- oder entriegelbar, so dass einer der beiden Arbeitsabschnitte wirksam wird. Das Bolzenteil kann eine durchgängige Axialbohrung zum Durchtritt eines Kirschner-Drahtes besitzen.

### Kurzbeschreibung der beigefügten Zeichnungen

Es zeigen:
- Figur 1A:: einen axialen Teilschnitt durch den Bolzen einer zu implantierenden Schraube;
- Figur 1B:: die Draufsicht auf den der Bolzenspitze gegenüberliegenden Bolzenschaft gemäss Figur 1A;
- Figur 1C:: einen axialen Teilschnitt durch den Kopf der Schraube gemäss Figur 1A;
- Figur 2A:: das auf einen Kirschner-Draht aufgesteckte Bolzenteil des Kombinations-Schraubwerkzeugs;
- Figur 2B:: einen axialen Schnitt durch das Kopfteil des Kombinations-Schraubwerkzeugs;
- Figur 2C:: das auf einen Kirschner-Draht aufgesteckte, zusammengesetzte Kombinations-Schraubwerkzeug im Teilschnitt in "Bolzenstellung";
- Figur 2D:: den Eingriff des Kombinations-Schraubwerkzeugs gemäss Figur 2C mit der Schraube in "Bolzenstellung";
- Figur 2E:: das Kombinations-Schraubwerkzeug gemäss Figur 2C in "Kopfstellung";
- Figur 2F:: den Eingriff des Kombinations-Schraubwerkzeugs gemäss Figur 2E mit der Schraube in "Kopfstellung";
- Figur 2G:: eine Schnittdarstellung der Mitnehmermechanik am Kombinations-Schraubwerkzeug gemäss Figur 2E auf der Linie B-B;
- Figur 3A:: eine Gewebeschutzhülse, in die das Kombinations-Schraubwerkzeug einsteckbar ist, mit Teilschnitten an der Fixierschraube und am Hülsenstück; und
- Figur 3B:: das Kombinations-Schraubwerkzeug in "Bolzenstellung" auf einen Kirschner-Draht aufgeschoben und in die Gewebeschutzhülse gemäss Figur 3A
eingesetzt.

### Ausführungsbeispiel

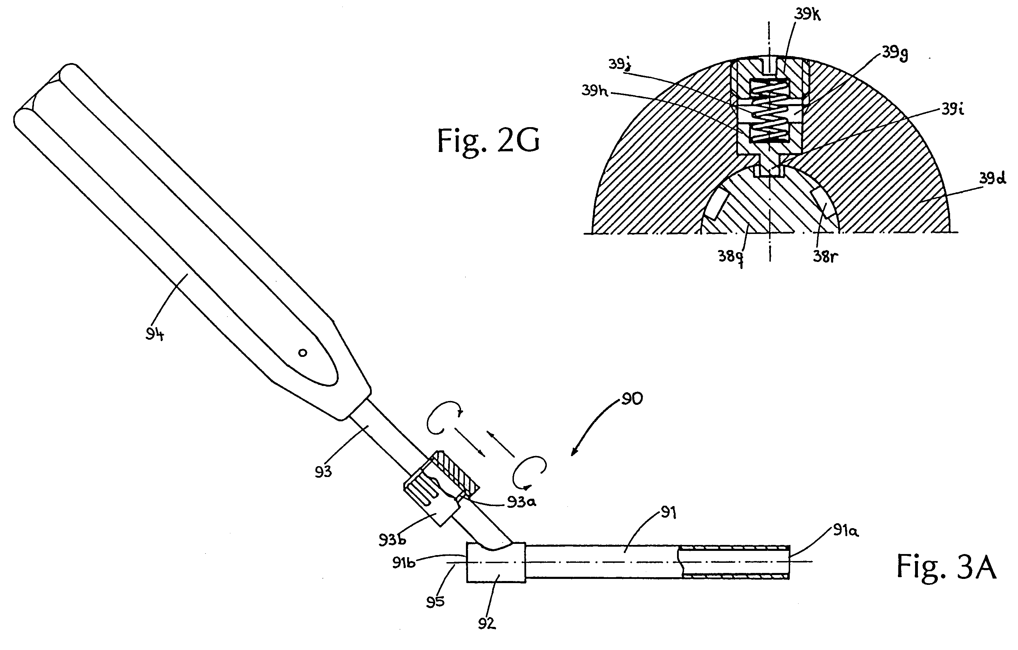

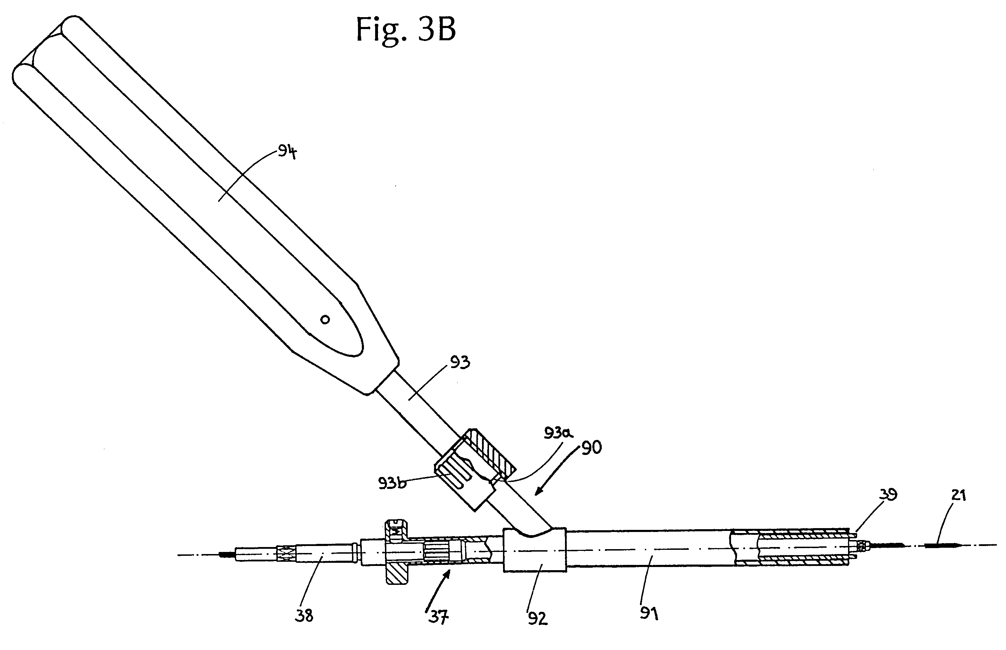

Mit Bezug auf die beiliegenden Zeichnungen erfolgt nachstehend die detaillierte Beschreibung eines bevorzugten Ausführungsbeispiels des erfindungsgemässen Schraubwerkzeugs. Zum besseren Verständnis wird zuerst auf die mit dem Schraubwerkzeug zu handhabende Schraube (siehe Figuren 1A bis 1C) eingegangen, die selbst nicht zum Erfindungsgegenstand gehört. Im Verlauf der Beschreibung wird auch die Gewebeschutzhülse (siehe Figuren 3A und 3B) erläutert - diese gehört selbst nicht zum Erfindungsgegenstand -, um das Zusammenwirken mit dem erfindungsgemässen Schraubwerkzeug darzulegen.

### Figuren 1A bis 1C

Der Bolzen **2** und der separate Kopf **3** (s. Figur 1C) sind miteinander verschraubbar und bilden die komplett zusammengesetzte Schraube. Eine theoretische, axiale Achse **4** verläuft durch den Bolzen **2**, den Kopf **3** und die zusammengesetzte Schraube **1**.

Der einstückige, hohlzylindrische Bolzen **2** - das Gewindeteil der Schraube **1** - weist ein sich hier oben befindliches erstes Ende **2a**; die Bolzenspitze und ein dieser abgewandtes zweites Ende **2b**, den Bolzenschaft, auf. Die Aussenfläche **2c** des Bolzens **2** hat einen bei dessen erstem Ende **2a** beginnenden, kurzen, zylindrischen Flächenabschnitt **2d**, an den sich ein erstes Gewinde **2e** anschliesst, dem zum zweiten Ende **2b** ein zweites Gewinde **2f** folgt. Der Durchmesser des Flächenabschnitts **2d** der Aussenfläche **2c** entspricht ungefähr dem Kerndurchmesser des ersten Gewindes **2e**. Der Aussendurchmesser des Gewindes **2e** bildet den grössten Durchmesser des Bolzens **2**. Der Aussendurchmesser des zweiten Gewindes **2f** ist kleiner als derjenige des ersten Gewindes **2e**.

Das erste Gewinde **2e** ist ein zum Einschrauben in einen Knochenteil bestimmtes Spezialgewinde mit einer relativ grossen Steigung. Das zweite Gewinde **2f** hingegen könnte ein normales metrisches Spitzgewinde oder mit wesentlich kleinerer Steigung sein. Das erste, längere Gewinde **2e** nimmt vorzugsweise mehr als 50% der Gesamtlänge des Bolzens **2** ein. Durch den Bolzen **2** erstreckt sich eine durchgängige Axialbohrung **2g**, die hauptsächlich von einem zylindrischen Hauptabschnitt **2h** gebildet wird und nur am zweiten Ende **2b** einen zur Achse **4** nicht-rotationssymmetrischen, vieleckförmigen, z.B. sechseckförmig konturierten Abschnitt **2i**, aufweist. Die Schlüsselweite des sechseckförmigen Abschnitts **2i** ist gleich dem Innendurchmesser des zylindrischen Hauptabschnitts **2h** oder geringfügig grösser. Der Bolzen **2** besitzt im Längsbereich des ersten Gewindes **2e** mehrere radiale Querbohrungen **2k**, welche in die Axialbohrung **2g** einmünden. Die Axialbohrung **2g** dient zum Durchstecken eines den Bolzen **2** führenden Kirschner-Drahtes und als Hohlraum für das spätere Einwachsen von Knochensubstanz, das durch die Querbohrungen **2k** geschieht.

Der im Prinzip zur Achse **4** rotationssymmetrische Kopf **3** - quasi die Mutter der Schraube **1** - setzt sich aus dem hülsenförmigen Hals **3b** und der Kopfpartie **3a** zusammen. Die Kopfpartie **3a** besitzt einen grösseren Durchmesser als der Hals **3b** und weist eine zylindrische Aussenfläche **3c** auf, an die sich eine zum Hals **3b** hin verjüngende, ringförmige Konusfläche **3d** anschliesst.

Durch den Kopf **3** erstreckt sich eine durchgängige Axialbohrung **3f**. Diese ist über ihre ganze Länge - abgesehen von zwei kurzen, konischen Ansenkungen - mit einem zum zweiten Gewinde **2f** des Bolzens **2** komplementären Innengewinde **3g** versehen, so dass die beiden Gewinde **2f** und **3g** miteinander verschraubbar sind. Somit ist der an sich separate Kopf **3** auf den Bolzen **2** aufschraubbar und auf diesem höhenvariabel positionierbar. Auf ihrer Stirnseite ist die Kopfpartie mit mehreren, hier vier Einschnitten **3h**, versehen, die sich von der zylindrischen Aussenfläche **3c** bis in die Axialbohrung **3f** erstrecken.

Ist der Kopf **3** auf den Bolzen **2** aufgeschraubt - es ergibt sich die komplett zusammengesetzte Schraube **1** -, entsteht ein sich durch die gesamte Schraube **1** erstreckender Axialdurchgang. Ragt der Kopf **3** der zusammengesetzten Schraube **1** über das zweite Ende **2b** des Bolzens **2** hinaus, so bildet die Axialbohrung **3f** des Kopfes **3** einen kurzen Abschnitt des Axialdurchgangs der Schraube **1**.

### Figuren 2A und 2B

Die bisher gebräuchlichen Schraubwerkzeuge waren monofunktionaler Art, d.h. man benötigte zum Eingriff in den Bolzen **2** und in den Kopf **3** zwei verschiedene, separate Schraubwerkzeuge. Jetzt wird ein bifunktionales Kombinations-Schraubwerkzeug, nachstehend verkürzt als Kombischrauber **37** bezeichnet, vorgestellt. Der Kombischrauber **37** besteht aus zwei teleskopisch ineinander steckbare Einheiten, nämlich dem stangenförmigen Bolzenteil **38** und dem hülsenförmigen Kopfteil **39**.

Das Bolzenteil **38** hat einen Schaft **38c** in Gestalt eines zylindrischen Abschnitts **38g** mit dem freien Schaftende **38d** und dem sich an letzteres anschliessenden, vieleckförmigen Abschnitt **38e**. Dem zylindrischen Abschnitt **38g** folgt ein Bund **38h**, der zuerst in eine Ringnut **38i** und dann über eine Anschlagfläche **38m** in einen dickeren, zylindrischen Abschnitt **38k** übergeht. Durch das gesamte Bolzenteil **38** erstreckt sich eine durchgängige Axialbohrung **38a**, um den Durchtritt eines Kirschner-Drahtes **21** zu ermöglichen. Der dickere, zylindrische Abschnitt **38k** endet in einer Anschlagfläche **38n**. Hinter dieser folgt ein zylindrischer Abschnitt **38p**, der dem Durchmesser des Abschnitts **38g** entspricht. An den Abschnitt **38p** schliesst sich nach einem harmonischen Übergang ein geringfügig verdickter Nutensektor **38q** an. An der Umfangsfläche des Nutensektors **38q** sind mehrere axial verlaufende und radial verteilte Nuten **38r** vorgesehen.

Hinter dem Nutensektor **38q** beginnt mit einem radial umlaufenden Hemmrand **38s** ein wiederum verdickter, kurzer, zylindrischer Abschnitt **38t**, der in einem harmonischen Übergang **38u** in einen durchmessereduzierten, zylindrischen und relativ langen Abschnitt **38v** übergeht. Der Abschnitt **38v** entspricht im Durchmesser dem Schaft **38c** sowie dem Abschnitt **38p** und erstreckt sich bis an den an der Spitze des Bolzenteils **38** liegenden, im Durchmesser reduzierten Arbeitsabschnitt **38b**. Zwischen dem Abschnitt **38v** und dem Arbeitsabschnitt **38b** ergibt sich somit eine kreisringförmige Anschlagfläche **38w**. Der Arbeitsabschnitt **38h** weist einen steckschlüsselartigen Vielkant **38x** auf, welcher dazu bestimmt ist, komplementär in den vieleckig konturierten Abschnitt **2i** des Bolzens **2** einzugreifen und die Drehbewegung zu übertragen.

Das Kopfteil **39** ist für die Verschraubung des Kopfes **3** vorgesehen und besteht im wesentlichen aus einem Hülsenstück **39c**, welches an einem Ende den Arbeitsabschnitt **39b** und am gegenüberliegenden Ende den Flansch **39d** aufweist. Der Arbeitsabschnitt **39b** wird aus mehreren, sich in axialer Richtung vom Hülsenstück **39c** fortsetzender Zähne **39x** gebildet. Er ist dazu bestimmt, komplementär in die am Kopf **3** stirnseitig vorhandenen Einschnitte **3h** einzugreifen und die Drehbewegung des Kopfteils **39** als Schraubbewegung auf den Kopf **3** zu übertragen.

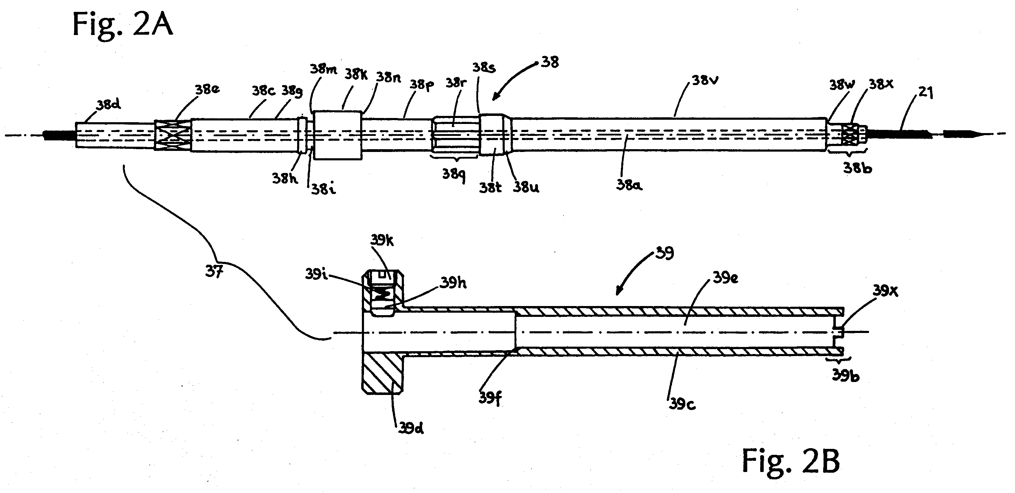

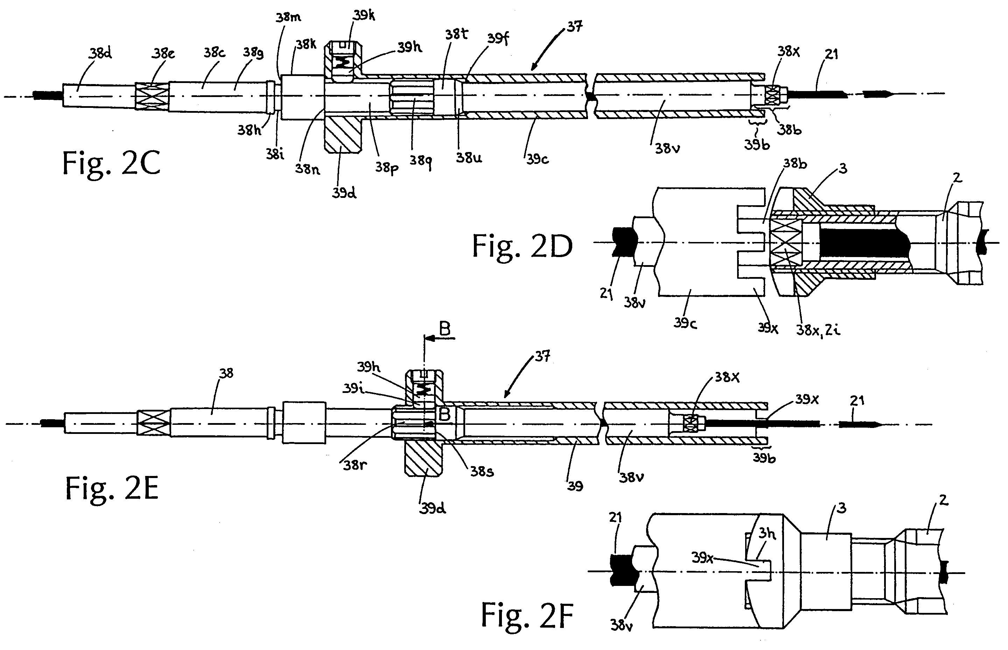

Durch das Hülsenstück **39c** verläuft ein Axialdurchgang **39e**, der sich vom Flansch **39d** kommend an einem Absatz **39f** einstufig verengt. Im Flansch **39d** ist eine radiale, sacklochförmige Bohrung **39g** vorgesehen (s. auch Figur 2G), von der sich ein verengter Durchstich **39h** hin zum Axialdurchgang **39e** erstreckt. In der Bohrung **39g** sitzt ein Rastelement **39h**, das mit seiner Rastnase **39i** den Durchstich **39h** durchdringt und so in den Axialdurchgang **39e** hineinragt. Das Rastelement **39h** wird von einer Feder **39j** in Richtung des Axialdurchgangs **39e** gedrückt, während sich die Feder **39j** an einer in die Bohrung **39g** eingesetzten Verschlussschraube **39k** abstützt.

### Figuren 2C und 2D

Hier ist der aus dem Bolzenteil **38** und dem Kopfteil **39** zusammengesetzte Kombischrauber **37** in der "Bolzenstellung" gezeigt, d.h. in dieser Stellung kann der Kombischrauber **37** als Schraubwerkzeug für einen Bolzen **2** verwendet werden, da der Arbeitsabschnitt **38b** aus dem Hülsenstück **39c** ausgefahren ist. Das Bolzenteil **38** ist maximal in das Kopfteil **39** eingeschoben, so dass die Anschlagfläche **38n** am Flansch **39d** anliegt und der Übergang **38u** nahe dem Absatz **38f** steht. Die Rastnase **39i** setzt mit leichtem Druck auf dem glatten Abschnitt **38p** auf und verursacht so beim Drehen des Bolzenteils **38** eine kaum spürbare Reibung.

Angesetzt an eine komplette Schraube **1**, bestehend aus dem Bolzen **2** und dem aufgeschraubten Kopf **3**, zeigt sich, dass der am Arbeitsabschnitt **38b** des Bolzenteils **38** vorhandene Vielkant **38x** in den Vielkantabschnitt **2i** des Bolzens **2** eingreift. Die Zähne **39x** des Kopfteils **39** hingegen stehen mit den im Kopf **3** vorhandenen Schlitzen **3h** ausser Eingriff.

### Figuren 2E bis 2G

Der Kombischrauber **37** befindet sich nun in der "Kopfstellung", d.h. in dieser Stellung ist der Kombischrauber **37** nur als Schraubwerkzeug für einen Kopf **3** in Aktion, da der Arbeitsabschnitt **38b** in das Hülsenstück zurückgezogen ist und somit der Arbeitsabschnitt **39b** zur Wirkung kommt. Um diese Stellung zu erreichen, zieht man das Bolzenteil **38** soweit aus dem Kopfteil **39** heraus, dass der Nutensektor **38q** unter das Rastelement **39h** gerät und die Rastnase **39i** in eine nahegelegene Nut **38r** gleitet. Das leichte weitere Herausziehen des Bolzenteils **38** aus dem Kopfteil **39** wird dadurch verhindert, dass die Rastnase **39i** gegen den Hemmrand **38s** stösst.

Bei Drehung des Bolzenteils **38** - eventuell mit einer angesetzten Drehvorrichtung -, dreht sich durch den Formschluss zwischen der Rastnase **39i** und der jeweils von dieser belegten Nut **38r** das Kopfteil **39** mit. Wird der Kombischrauber **37** in der "Kopfstellung" an eine komplette Schraube **1** angesetzt, tritt nur das Kopfteil **39** in Aktion, indem seine Zähne **39x** in die Einschnitte **3h** am Kopf **3** eingreifen, während der zurückgezogene Vielkant **38x** des Bolzenteils **38** nicht zur Wirkung kommt.

Der mit der Axialbohrung **38a** versehene Kombischrauber **37** und die in der Schraube **1** vorhandenen Axialbohrungen **2g,3f** ermöglichen, dass der Kirschner-Draht **21** während der Operation dauernd im Knochen bleiben kann, bis der Bolzen **2** eingeschraubt und auch der Kopf **3** festgeschraubt sind. Durch die Schraube **1** werden die zu verbindenden Knochenfragmente von der erzeugten Druckkraft gegeneinander gedrückt. Hat man den Kirschner-Draht **21** tatsächlich bis nach dem Festschrauben des Kopfes **3** noch im Knochen belassen, wird dieser nun entfernt und die Operationswunde geschlossen.

Will man den Kombischrauber **37**, z.B. zu Reinigungszwecken, auseinandernehmen, ist es lediglich erforderlich, das Bolzenteil **38** gänzlich aus dem Kopfteil **39** herauszuziehen. Unter Aufwendung einer gewissen Zugkraft überspringt die Rastnase **39i** den Hemmrand **38s.**

### Figur 3A

Um sämtliche in Betracht kommenden Operationsvorgänge minimal-invasiv ausführen zu können, benutzt man eine Gewebeschutzhülse **90,** durch welche die einzelnen Operationsinstrumente hindurchgeführt werden. Die Gewebeschutzhülse **90** wird in einen kurzen Schnitt im Hals eingesetzt. Somit entfällt das Auseinanderziehen der Operationswunde mit Haken und das häufige Ein- und Ausfahren mit Instrumenten durch diese Wunde. Bereits das Einsetzen des Kirschner-Drahtes **21** kann mittels einer Bohrlehre über die Gewebeschutzhülse **90** geschehen.

Hauptbestandteile der Gewebeschutzhülse **90** sind das Kanalrohr **91** - die eigentliche Gewebeschutzhülse -, die Halterung **92**, der Verbindungssteg **93** mit dem daran befindlichen Stellmittel **93a** und dem Fixierorgan **93b** sowie der Griff **94**. Das Kanalrohr **91** wird von einem Rohrstück gebildet, welches die leichtgängige aber möglichst spielfreie Aufnahme der verschiedenen Instrumente gestattet. Zuvorderst besitzt die Führungshülse die Mündungsöffnung **91a** und zuhinterst die Eintrittsöffnung **91b**. Nahe der Eintrittsöffnung **91b** setzt die Halterung **92** drehfest an die Führungshülse **92** an. Hier umfasst die Halterung **92** das Kanalrohr **91** manschettenartig. Von der Halterung **92** erstreckt sich im stumpfen Winkel zur Mittelachse **95** der Führungshülse **92** der Verbindungssteg **93**, an den andererseits der Griff **94** angesetzt ist.

### Figur 3B

Wie die Instrumente zur Herstellung des Bohrlochs **15** in der Gewebeschutzhülse **90** geführt und gehaltert werden können, ist es auch möglich, den Kombischrauber **37** in die Gewebeschutzhülse **90** einzusetzen und so via die Gewebeschutzhülse **90** mit dem bifunktionalen Kombinations-Schraubwerkzeug **27** in die zu verbindenden Knochenfragmente eine Schraube **1** zu implantieren, d.h. einzuführen, festzuschrauben oder zu lösen.

## Patentansprüche

1. Schraubwerkzeug (**37**) für eine Schraube (**1**), insbesondere zum Zusammenhalten von zwei Knochenfragmenten mit einem Bolzen (**2**) und einem darauf aufschraubbaren Kopf (**3**), wobei
a) der Bolzen (**2**) aufweist:
- eine durchgängige Axialbohrung (**2g**), und
- an dem Ende (**2b**), welches zur Verschraubung mit dem Kopf (**3**) dient, einen Aussengewindeabschnitt (**2f**), und
- innerhalb der Axialbohrung (**2g**) als Mitnehmermittel für den Eingriff des Schraubwerkzeugs (**37**) einen zur Achse (**4**) nicht-rotationssymmetrischen, vieleckförmigen Abschnitt (**2i**); und
b) der Kopf (**3**) aufweist:
- eine durchgängige Axialbohrung (**3f**) mit einem Innengewinde (**3g**), das zum Verschrauben mit dem Aussengewindeabschnitt (**2f**) bestimmt ist, und
- Einschnitte (**3h**) als Mitnehmermittel für den Eingriff des Schraubwerkzeugs (**37**);
c) der sich durch die komplette Schraube (**1**) ziehende Axialdurchgang (**2g,3f**) zum Durchtritt eines Kirschner-Drahtes (**21**) ausgelegt ist, dadurch gekennzeichnet, dass
d) das Schraubwerkzeug (**37**) als bifunktionales Kombinations-Schraubwerkzeug aus einem Bolzenteil (**38**) und einem Kopfteil (**39**) besteht, die alternativ in Funktion setzbar sind;
e) das Bolzenteil (**38**) und das Kopfteil (**39**) verschieden konturierte Arbeitsabschnitte (**38b,39b**) aufweisen, die dazu bestimmt sind, in den Bolzen (**2**) bzw. den Kopf (**3**) formschlüssig einzugreifen und die Drehbewegung des Schraubwerkzeugs (**37**) auf diese zu übertragen;
f) das Bolzenteil (**38**) teleskopisch in das Kopfteil (**39**) einsetzbar ist;
g) durch Relativverschiebung zwischen dem Bolzenteil (**38**) und dem Kopfteil (**39**) einer der beiden Arbeitsabschnitte (**38b,39b**) wirksam wird; und
h) zugleich mit der Relativverschiebung die Mitnehmerfunktion zwischen dem Bolzenteil (**38**) und dem Kopfteil (**39**) wahlweise herstellbar oder entriegelbar ist.

2. Schraubwerkzeug nach Anspruch 1, dadurch gekennzeichnet, dass
a) das Bolzenteil (**38**) eine durchgängige Axialbohrung (**38a**) zum Durchtritt des Kirschner-Drahtes (**21**) besitzt und im Verlauf seines Schafts (**38c**) einen Nutensektor (**38q**) mit zumindest einer axial ausgerichteten, umfangsseitig angeordneten Nut (**38r**) aufweist; und
b) am Kopfteil (**39**) ein die Mitnehmerfunktion bewirkendes Rastelement (**39h**) vorgesehen ist, welches mit der Relativverschiebung zwischen dem Bolzenteil (**38**) und dem Kopfteil (**39**) mit der Nut (**38r**) am Bolzenteil (**38**) in Eingriff gebracht werden kann.

3. Schraubwerkzeug nach Anspruch 1 oder 2, dadurch gekennzeichnet, dass
a) am Bolzenteil (**38**) im Verlauf seines Schafts (**38c**) ein verdickter, zylindrischer Abschnitt (**38k**) vorgesehen ist, dessen dem Arbeitsabschnitt (**38b**) zugewandte kreisringförmige Stirnseite eine Anschlagfläche (**38n**) darstellt, welche die Einschubtiefe des Bolzenteils (**38**) in das Kopfteil (**39**) begrenzt;
b) am Kopfteil (**39**), an dem dem Arbeitsabschnitt (**39b**) gegenüberliegenden Ende, ein Flansch (**39d**) angeordnet ist, in dem unter Spannung einer Feder (**39j**) das Rastelement (**39h**) gehalten ist, von dem eine Rastnase (**39i**) - zum Eingriff mit der Nut (**38r**) bestimmt - in den Axialdurchgang (**39e**) des Hülsenstücks (**39c**) des Kopfteils (**39**) ragt; und
c) die Arbeitsabschnitte (**38b,39b**) jeweils komplementäre Konturen (**38x,39x**) zu den Eingriffskonturen (**2i,3h**) des Bolzens (**2**) bzw. des Kopfes (**3**) besitzen.

4. Schraubwerkzeug nach einem der Ansprüche 1 bis 3 dadurch gekennzeichnet, dass dieses in das Kanalrohr (**91**) einer zum minimal-invasiven Einbringen von Operationsinstrumenten vorgesehenen Gewebeschutzhülse (**90**) einsetzbar und zugleich mit einer Drehvorrichtung koppelbar ist.

## Claims

1. Screwdriver (37) for a screw (1), in particular for holding together two bone fragments with a bolt (2) and a head (3) which can be screwed onto the latter, in which
a) the bolt (2) has:
- a continuous axial bore (2g), and
- an externally threaded section (2f) at the end (2b) which serves for the screw connection to the head (3), and
- a polygonal section (2i) which is not rotationally symmetrical with respect to the axis (4) and is situated inside the axial bore (2g) as catch means for the screwdriver (37) to engage in; and
b) the head (3) has:
- a continuous axial bore (3f) with an internal screw thread (3g), which is intended to be screwed onto the externally threaded section (2f), and
- notches (3h) as catch means for the screwdriver (37) to engage in;
c) the axial passage (2g, 3f) which runs through the complete screw (1) is designed for the passage of a Kirschner's wire (21), characterized in that
d) the screwdriver (37), as a dual-function combination screwdriver, comprises a bolt part (38) and a head part (39), which can be put into use as alternatives to one another;
e) the bolt part (38) and the head part (39) have differently contoured working sections (38b, 39b), which are intended to engage in a form-fitting manner in the bolt (2) or the head (3) and to transmit the rotary movement of the screwdriver (37) to these components;
f) the bolt part (38) can be inserted telescopically into the head part (39);
g) one of the two working sections (38b, 39b) is made active by means of a relative displacement between the bolt part (38) and the head part (39); and
h) at the same time as the relative displacement, the catch function between the bolt part (38) and the head part (39) can be produced or disengaged as desired.

2. Screwdriver according to Claim 1, characterized in that
a) the bolt part (38) has a continuous axial bore (38a) for the passage of the Kirschner's wire (21) and, over the course of its shank (38c), has a groove sector (38q) with at least one axially aligned, circumferentially arranged groove (38r); and
b) a latching element (39h) is provided on the head part (39), which latching element effects the catch function and, by means of the relative displacement between the bolt part (38) and the head part (39), can be brought into engagement with the groove (38r) on the bolt part (38).

3. Screwdriver according to Claim 1 or 2, characterized in that
a) over the course of the shank (38c) of the bolt part (38) there is provided a thickened, cylindrical section (38k), the annular end face of which, directed towards the working section (38b), forming a stop face (38n) which limits the penetration depth of the bolt part (38) into the head part (39);
b) on the head part (39), at the end situated opposite to the working section (39b), there is arranged a flange (39d), in which the latching element (39h) is held under loading from a spring (39j), from which latching element a latching lug (39i) - destined to engage with the groove (38r) - projects into the axial passage (39e) of the sleeve part (39c) of the head part (39); and
c) the working sections (38b, 39b) each have contours (38x, 39x) which are complementary to the engagement contours (2i, 3h) of the bolt (2) and the head (3), respectively.

4. Screwdriver according to one of Claims 1 to 3, characterized in that it can be inserted into the channel tube (91) of a tissue-protection sleeve (90), which is provided for the minimally invasive introduction of surgical instruments, and at the same time can be coupled to a turning device.

## Revendications

1. Outil de vissage (37) pour un boulon (1) destiné notamment à maintenir ensemble deux fragments d'os et comprenant une vis (2) et une tête (3) pouvant être vissée sur cette vis,
a) la vis (2) présentant :
- un perçage axial traversant (2g), et
- à l'extrémité (2b) qui sert à l'assemblage vissé avec la tête (3), un tronçon (2f) à filetage extérieur, et
- à l'intérieur du perçage axial (2g), comme moyen d'entraînement pour l'engagement de l'outil de vissage (37), un tronçon polygonal (2i) sans symétrie de révolution par rapport à l'axe (4) ; et
b) la tête (3) présentant :
- un perçage axial traversant (3f), pourvu d'un filetage intérieur (3g) destiné à l'assemblage vissé avec le tronçon (2f) à filetage extérieur, et
- des encoches (3h) comme moyen d'entraînement pour l'engagement de l'outil de vissage (37) ;
c) le passage axial (2g, 3f) s'étendant à travers le boulon complet (1) étant conçu pour le passage d'une broche de Kirschner (21),
**caractérisé** en ce que
d) l'outil de vissage (37), est constitué, en tant qu'outil de vissage combiné à double fonction, d'une partie pour vis (38) et d'une partie pour tête (39) qui peuvent être alternativement mises en service ;
e) la partie pour vis (38) et la partie pour tête (39) présentent des tronçons de travail (38b, 39b) de contours différents, qui sont destinés à s'engager respectivement, par complémentarité de forme, dans la vis (2) ou la tête (3), et leur transmettre le mouvement de rotation de l'outil de vissage (37) ;
f) la partie pour vis (38) peut être insérée télescopiquement dans la partie pour tête (39) ;
g) un des deux tronçons de travail (38b, 39b) est rendu actif par un déplacement relatif entre la partie pour vis (38) et la partie pour tête (39) ; et
h) en même temps que le déplacement relatif, la fonction d'entraînement entre la partie pour vis (38) et la partie pour tête (39) peut être, au choix, réalisée ou déverrouillée.

2. Outil de vissage selon la revendication 1, **caractérisé** en ce que
a) la partie pour vis (38) possède un perçage axial traversant (38a) pour le passage de la broche de Kirschner (21) et présente, dans le développement de sa queue (38c), un secteur rainuré (38q) pourvu d'au moins une rainure périphérique (38r) orientée axialement ; et
b) un élément de crantage (39h) produisant la fonction d'entraînement est prévu sur la partie pour tête (39), élément qui, par le déplacement relatif entre la partie pour vis (38) et la partie pour tête (39), peut être amené en engagement avec la rainure (38r) prévue sur la partie pour vis (38).

3. Outil de vissage selon la revendication 1 ou 2, **caractérisé** en ce que
a) un tronçon cylindrique épaissi (38k) est prévu sur la partie pour vis (38) dans le développement de sa queue (38c), tronçon dont la face frontale annulaire tournée vers le tronçon de travail (38b) constitue une face de butée (38n) qui limite la profondeur d'enfoncement de la partie pour vis (38) dans la partie pour tête (39) ;
b) un collet (39d) est disposé sur la partie pour tête (39), à l'extrémité opposée au tronçon de travail (39b), collet dans lequel l'élément de crantage (39h) est maintenu sous la contrainte d'un ressort (39j), un ergot de crantage (39i), destiné à s'engager avec la rainure (38r), dépassant de l'élément de crantage (39h) pour faire saillie dans le passage axial (39e) du tronçon de manchon (39c) de la partie pour tête (39) ; et
c) les tronçons de travail (38b, 39b) présentent des contours (38x, 39x) respectivement complémentaires des contours d'engagement (2i, 3h) de la vis (2) et de la tête (3).

4. Outil de vissage selon l'une quelconque des revendications 1 à 3, **caractérisé** en ce que cet outil peut être inséré dans le tube de passage (91) d'un manchon protecteur de tissu (90) prévu pour l'introduction d'instruments opératoires avec une invasion minimale, et il peut être en même temps accouplé à un dispositif de rotation.
